# EUROPEAN PATENT APPLICATION

(11) **EP 1 138 292 A1**
(43) Date of publication of application: **04.10.2001**
(21) Application number: 00201099.9
(22) Date of filing: 27.03.2000
(51) Int. Cl.: A61F 11/00

(54) **Instrument for cleaning body cavities, typically the acoustic meatus**

(71) Applicant: Federzoni, Luca, 41028 Serramazzoni (Modena) (IT)
(72) Inventor: Federzoni, Luca, 41028 Serramazzoni (Modena) (IT)
(74) Representative: Corradini, Corrado

(57) **Abstract**

The instrument comprises a gripping and manoeuvring shank (10) and at least one internally hollow part (2) joined to that end of the shank (10) which is to penetrate into the body cavity and having a thin flexible wall (20). The wall (20) has at least one relatively thin edge (21) arranged to scrape wax from the inner surfaces of the cavity; the hollow part (2) is able to deform elastically against the inner surfaces of the cavity during normal use. The hollow part (2) comprises at least one narrow thin blade (20) of elastically flexible material bent back on itself to form a round curved line which projects in the longitudinal direction beyond the end of the shank (10) and has a transverse dimension (D) much greater than the thickness of the shank (10).

## Description

This invention relates to a small instrument for cleaning cavities of the human body.

A typical but not exclusive use of the invention is in the removal od wax from the external acoustic meatus.

With particular reference to said acoustic meatus, this is usually cleaned using disposable instruments comprising a small rigid or non-rigid rod, usually of synthetic material, having at one end a substantially oval cotton wool pad of a size enabling it to be inserted into the acoustic meatus.

The widespread use of such instruments has proved problematic because they do not always achieve the desired result, ie the cleaning of the cavity, but instead can aggravate the existing situation.

In this respect, the insertion of said pad, particularly into an acoustic meatus, often generates an undesirable thrust against at least a part of the wax mass present therein, with the result that this becomes even more compacted and forced inwards.

This is obviously annoying for the person concerned, and moreover if the wax mass is rather large and dense the said thrust can result in complete occlusion of the acoustic meatus.

A further drawback of said instruments is that they can also damage the tympanic membrane, which happens often.

Hence in this sector there is a deeply felt need for means which obviate these drawbacks.

The main object of this invention is to satisfy this requirement within the context of a constructionally simple, rational, reliable and safe solution.

This and further objects are attained by the invention as characterised in the claims.

The invention is described in detail hereinafter with the aid of the accompanying figures, which show a non-exclusive embodiment thereof by way of non-limiting example.

Figure 1 is a perspective view showing a first embodiment of the invention.

Figure 2 is an axial section through the instrument of Figure 1.

Figure 3 is a schematic view showing the instrument of the invention applied to an acoustic meatus.

Figure 4A is an enlarged front view of the hollow part of Figure 1.

Figure 4B is a side view of Figure 4A.

Figure 5A is a side view of a second embodiment of the hollow part.

Figure 5B is a plan view of Figure 5A from above.

Figure 6 is a perspective view of a third embodiment of the hollow part.

Figure 7 is a perspective view of a fourth embodiment of the hollow part.

Figure 8 is a perspective view of a fifth embodiment of the hollow part.

Figure 9 is a perspective view of a sixth embodiment of the hollow part.

Figure 10 is a perspective view of a seventh embodiment of the hollow part.

Figure 11 is a perspective view of the entire instrument in a further embodiment.

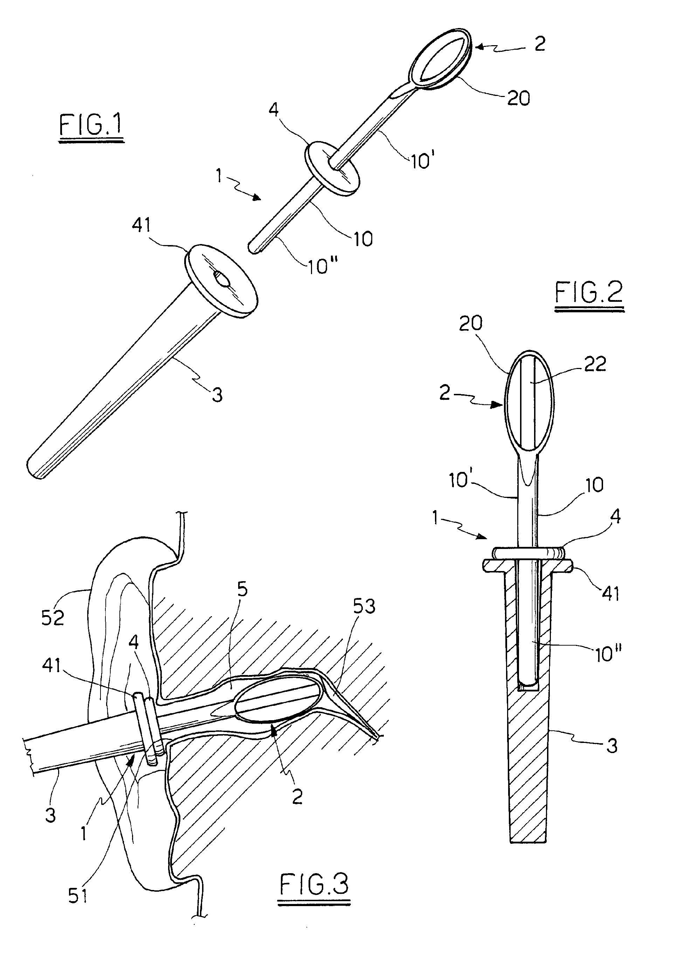

From said figures, and in particular Figures 1-3, it can be seen that the invention comprises a gripping and manoeuvring shank, composed in particular of a straight rod 10 having a diameter of 1-4 mm and a length of a few centimetres.

To the upper end of the shank 10, namely that end which is to penetrate into the cavity, there is joined an internally hollow part 2 having a thin flexible wall 20 possessing at least one relatively thin edge 20 arranged to scrape wax from the inner surfaces of the body cavity (in particular of the acoustic meatus 5) and able to deform elastically against the inner surfaces of the body cavity during normal use.

Preferably, said wall 20 of said hollow part 2 is defined by at least one narrow thin blade of elastically flexible material bent back on itself to form a round line which curves towards itself to form a loop projecting in the longitudinal direction beyond the end of the shank 10 and having a transverse dimension (D) much greater than the thickness of the shank 10. The blade 20 has two opposing edges 21, namely a front and a rear edge, both able to scrape wax from the inner surfaces of the body cavity.

Preferably, the plane in which the blade 20 is bent is a plane substantially passing through the axis of the shank 10, the major axis of the curve being substantially coaxial with the shank itself.

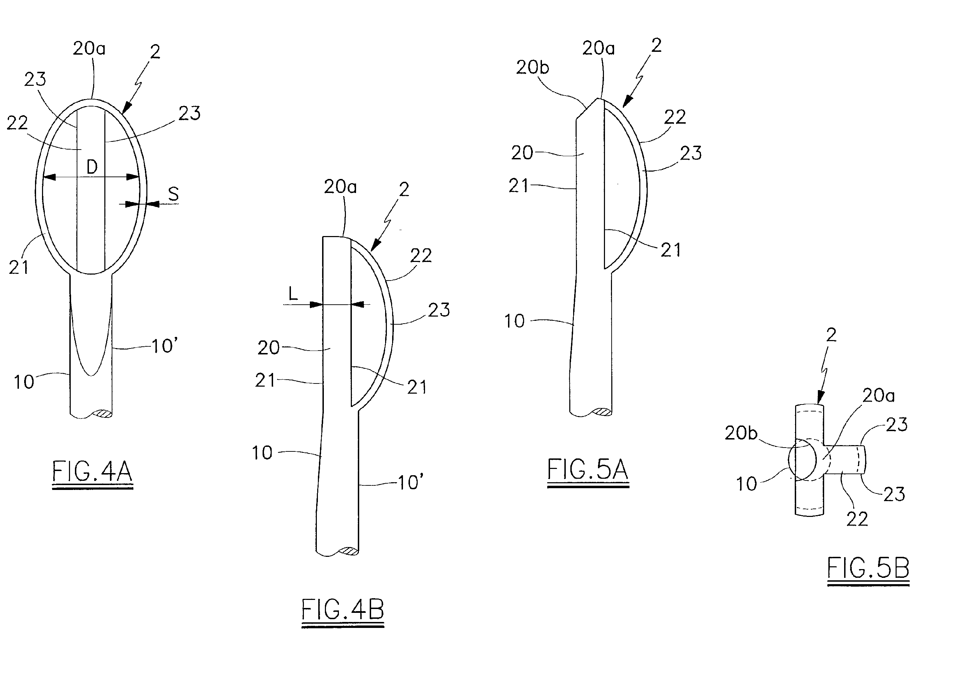

In particular, the blade 20 consists of a thin layer of atoxic synthetic resin moulded in one piece with the shank 10. Its width, ie that dimension (indicated by L in Figure 4B) which extends perpendicular to the axial bending plane of the blade 20, is 2-4 mm, its thickness, ie that dimension (indicated by S in Figure 4A) which extends within said plane of the edges 21, being considerably less than its width (of the order of some tenths of a millimetre, say 0.3-0.4 mm).

In the embodiment shown in Figures 1-3, 4A and 4B, the flexible blade 20 is bent to form an elliptical (or circular) loop and both its ends are joined to the end of the shank 10.

The general shape of the hollow part 2 is that of a spoon. It comprises in particular a second blade 22 bent into an open arc (equal to one half that of the blade 20) having its lower end joined to the upper end of the shank 10 and its other end joined to the top 20a of the first blade 20. The second blade 22 lies in the axial plane perpendicular to that in which the first blade 20 lies and possesses two edges 23 also able to scrape wax from the inner surfaces of the acoustic meatus.

Overall, the two blades 20 and 22 together form a spoon with two segmental apertures 29.

In the version suitable for cleaning the acoustic meatus 5, and shown in Figures 1-3, the hollow part 2 preferably has a transverse dimension (indicated by D in Figure 4A) greater than the transverse dimension of the acoustic meatus 5. For example, said dimension D can be 6-10 mm.

The lower part 10" of the shank 10 is gripped by two fingers to manoeuvre the instrument 1 when in use.

To improve gripping comfort, a gripping element 3 (shown in Figures 1-3) can be provided having an elongate shape and a circular cross-section with its diameter greater than the diameter of the shank 10. The lower portion 10" of the shank is inserted into the gripping element 3, to be removably secured to it. For this, the gripping element 3 possesses an axial cavity 31 for receiving the lower portion 10" of the shank by slight forcing, so as to be removably secured to said portion 10".

Again in the version suitable for cleaning the acoustic meatus, a radial stop element 4 in the form of a circular plate is advantageously provided on the shank 10 in a plane perpendicular to the axis of the shank 10 and has a maximum dimension greater than the diameter of the entrance 51 to the acoustic meatus at the centre of the auricle 52. The stop element 4 separates the lower portion 10" from the upper portion 10' of the shank 10, its purpose being to prevent penetration of the lower portion 10" into the acoustic meatus 5.

The upper portion 10' of the shank lying above the radial element 4 is shorter than the length of the acoustic meatus 5 measured between its entrance 51 and the tympanic membrane 53, the lower shank portion 10" below the radial element 4 being gripped by the fingers to support and manoeuvre the described instrument 1.

When in use, the instrument 1 can be gripped with two fingers either directly by the lower shank portion 10", or more comfortably by the gripping element 3, after forcibly inserting the lower portion 10" into the cavity 31.

The hollow part 2 is then inserted into the acoustic meatus 5 and is rotated therein by the gripping fingers about the axis of the shank 10.

During rotation, both edges 21 of the blade 20 slide against the inner surface of the acoustic meatus 6, to separate the wax therefrom and collect it in the interior of the spoon defined by the hollow part 2. The blade 2 operates as a scraper, its width L (2-4 mm) being such as to render it sufficiently rigid against thrusts undergone by the edges 21 in scraping the wax away from the surface of the acoustic meatus (these thrusts being perpendicular to the bending plane of the blade 20). This is advantageous for the efficiency of the scraping action.

At the same time, the thickness S of the blade 20, being relatively small, makes this very deformable within the plane in which the edges 21 lie, with elastic deformation (because of the nature of the constituent material of the blade). This means that the entire part 2, and in particular the edges 21, deform to adapt and adhere to the inner surface against which the edges act. This fact increases the effectiveness and intensity of the scraping action.

The second blade 22 also acts as a scraper element, via its edges 23, to remove wax in the same manner as the blade 20.

This capacity to deform elastically against the inner surfaces of the cavity during normal use renders the action of the hollow part 2 delicate and without danger to the ear. At the same time the hollow part 2 has an unusual capacity to adapt to the various shapes which the inner surfaces of the acoustic meatus can assume.

Finally by virtue of the spoon form, the scraped wax accumulates within the region defined by the curve of the part 2 and is extracted from the acoustic meatus together with the instrument 1.

The said method of operation is used whether a mass of fairly soft material (such as wax) or a mass of fairly consistent material is present, this having been confirmed by specific tests carried out on prototypes in accordance with the teachings of the invention.

The radial element 4 abuts against the entrance 51 to prevent the instrument 1 penetrating further into the meatus 5. The length of the upper rod portion 10'is such as to prevent the shank reaching the membrane 53, so preventing any danger of damage to the membrane, which is notoriously very delicate.

Preferably the lower portion 10", of the shank is also shorter than the distance between the entrance 51 and the membrane, so preventing said lower portion, should it be inserted into the meatus 5, from reaching and damaging the membrane 53.

If the gripping element 3 is used, the stop element can be positioned on the upper end of the gripping element, in the form of a circular plate 41 lying in a plane perpendicular to the axis of the gripping element 3 and having a maximum dimension greater than the dimension of the entrance 51 to the acoustic meatus. In this case the shank 10 can be without the radial element 4.

The instrument 1 can be gripped and manoeuvred by the gripper element 3, which allows more comfortable gripping of the instrument 1 by the fingers. However, alternatively the user can directly grip the lower part 10" of the shank.

The shank 10 can be provided with small longitudinal ribs, or other equivalent roughness (not shown in the figures), to improve the grip of the fingers when the instrument 1 is to be rotated about the axis of the rod 10. Alternatively the rod 10 can have a polygonal cross-section.

The instrument 1 can also be conveniently used for other body cavities, for example the nasal cavities.

The embodiments shown in Figures 5A to 10 relate to variations limited to the hollow part 2.

In the embodiment shown in Figures 5A and 5B the blade 20 has a small portion removed at its top 20a along a cutting plane T lying at 20-70 degrees to the longitudinal axis, to form an arcuate scraper edge 20b. The edge 20b defines a scraper edge which, when the instrument 1 is rotated about the axis of the shank 10, acts at the top 20a of the part 2, and is particularly suitable for fairly consistent wax masses.

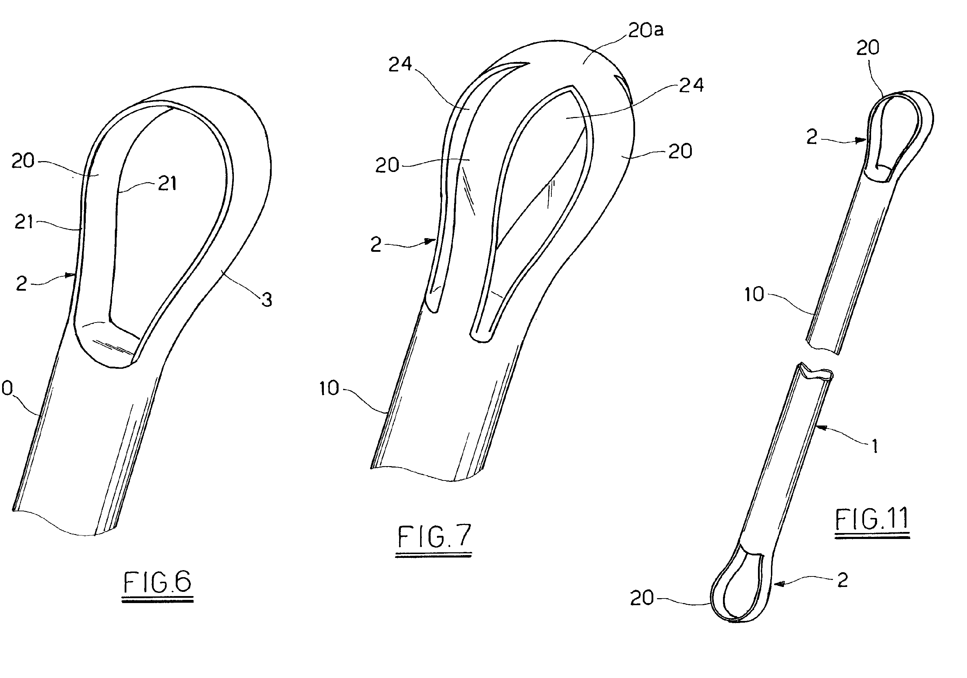

The embodiment shown in Figure 6 differs from the preceding in that it lacks the second blade 22, only the blade 20 being present.

In the embodiment shown in Figure 7, the hollow part 2 comprises two substantially identical narrow thin blades 20 of elastically flexible material, bent into a loop and having their ends all joined to the upper end of the shank 10. The blades 20 intersect at the top 20a, where they are joined together. The planes of curvature of the blades 20 are mutually perpendicular axial planes, to provide a bulb shape comprising four longitudinal apertures 24 extending from the end of the shank 10 and converging at the top 20a.

A small aperture, for example circular, can be provided in the top 20a, to define a scraper edge acting at a point, similar to the aforedescribed scraper edge 20b.

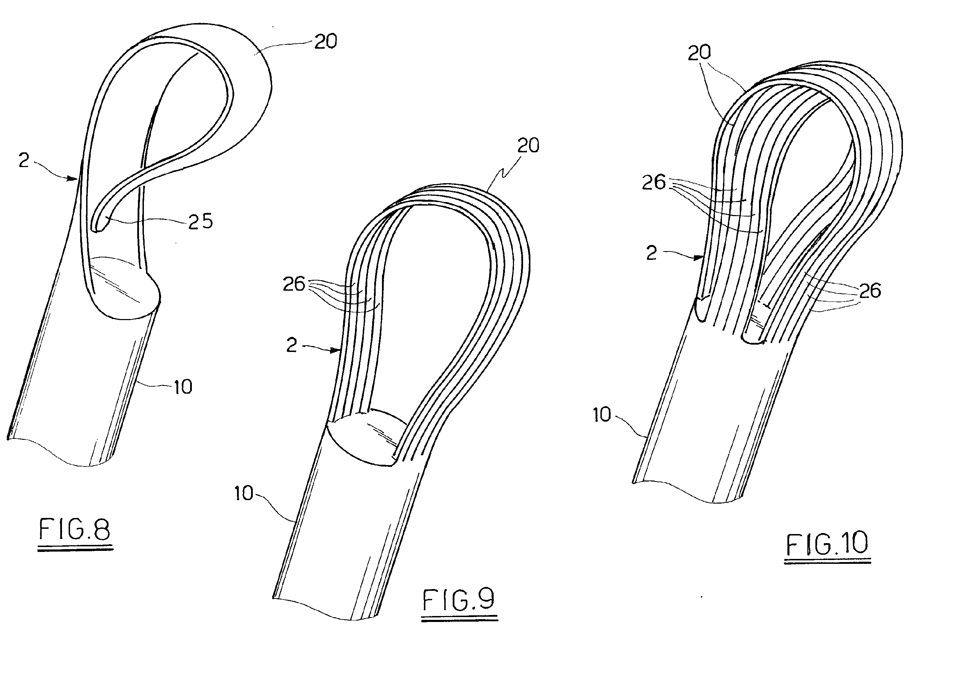

In the embodiment shown in Figure 8, the hollow part 2 consists of a tapering blade extending from the end of the shank 10 and bent back on itself, in an axial plane, to form a loop which is not completely closed.

The free end part 25 is bent in the opposite direction and elastically rests against the opposing part of the blade 20.

In the embodiment shown in Figures 9 and 10, the blades 20 forming the hollow part 2 each consist of a plurality of adjacent thin strips 26, typically obtained by cutting a thin blade similar to that provided in the embodiments shown in the preceding figures.

In the embodiment shown in Figure 11, two hollow parts 2 are provided, one joined to one end of the shank 10 and the other to its other end.

The invention can be produced by moulding synthetic resin at very low cost, and is typically of the disposable type for once-only use.

Numerous modifications of a practical and applicational nature can be made to the invention, but without leaving the scope of the inventive idea as claimed below.

## Claims

1. An instrument for cleaning body cavities, such as the acoustic meatus, comprising a gripping and manoeuvring shank (10), **characterised by** comprising at least one internally hollow part (2) joined to that end of the shank (10) which is to penetrate into the body cavity and having a thin flexible wall (20) with at least one relatively thin edge (21) arranged to scrape wax from the inner surfaces of the cavity, said hollow part (2) being able to deform elastically against the inner surfaces of the cavity during normal use.

2. An instrument as claimed in claim 1, **characterised in that** said hollow part (2) comprises at least one narrow thin blade (20) of elastically flexible material bent back on itself to form a round curved line which projects in the longitudinal direction beyond the end of the shank (10) and has a transverse dimension (D) much greater than the thickness of the shank (10).

3. An instrument as claimed in claim 2, **characterised in that** the ends of said blade (20) are both joined to the end of the shank (10).

4. An instrument as claimed in claim 2, **characterised in that** said blade (20) has a width (L) from 2 to 4 mm, and a thickness (S) of the order of some tenths of a millimetre.

5. An instrument as claimed in claim 2, **characterised in that** said hollow part (2) has the overall shape of a spoon.

6. An instrument as claimed in claim 5, **characterised in that** the hollow part (2) comprises a second blade (22) which has its axial plane perpendicular to that in which the first blade (20) lies and is shaped as an open arc with one end joined to the end of the shank (10) and its other end joined to the top (20a) of the first blade (20).

7. An instrument as claimed in claim 2, **characterised in that** the hollow part (2) comprises two narrow thin blades (20) of elastically deformable material bent as a loop, the planes in which the respective loops lie being mutually perpendicular axial planes, to form a bulb shape having four longitudinal apertures (24).

8. An instrument as claimed in claim 2, **characterised in that** the hollow part (2) consists of a tapering blade (25) extending from the end of the shank (10) and bent back on itself, in an axial plane, to form a loop which is not completely closed.

9. An instrument as claimed in claim 2, **characterised in that** said at least one blade (20) consists of a plurality of thin adjacent strips (26).

10. An instrument as claimed in claim 2, **characterised in that** said blade (20) has a portion removed at its top along a cutting plane (T) inclined at 20-70 degrees to the longitudinal axis, to form an arcuate scraper edge (20b).

11. An instrument as claimed in claim 2, for cleaning the acoustic meatus, **characterised in that** hollow part has a transverse dimension greater than the transverse dimension of the acoustic meatus (5).

12. An instrument as claimed in claim 1, for cleaning the acoustic meatus, **characterised by** comprising, positioned on the shank (10), a radial stop element (4) of maximum dimension greater than the dimension of the entrance (51) to the acoustic meatus (5), to prevent that shank portion (10") below the stop element (4) from penetrating into the acoustic meatus (5).

13. An instrument as claimed in claim 12, **characterised in that** that shank portion (10') above the radial element (4) is shorter than the length of the acoustic meatus (5) measured between its entrance (51) and the tympanic membrane (53).

14. An instrument as claimed in claim 12, **characterised in that** that shank portion (10") below the radial element (4) is gripped by the fingers in order to support and manoeuvre the instrument.

15. An instrument as claimed in claim 1, **characterised by** comprising a gripping element (3) of elongate shape which receives the lower portion (10") of the shank by insertion, to be removably secured to it.

16. An instrument as claimed in claim 15, **characterised in that** the gripping element (3) possesses an axial cavity (31) to receive the lower portion (10") of the shank as an exact fit, to be removably secured to it.

17. An instrument as claimed in claims 12 and 15, **characterised in that** said stop element (4) is positioned on the upper end of the gripping element.

18. An instrument as claimed in claim 1, **characterised in that** said hollow part (2) consists of a thin layer of atoxic synthetic resin moulded in one piece with the shank (10).
